# EUROPÄISCHE PATENTSCHRIFT

(11) **EP 3 294 168 B1**
(45) Veröffentlichungstag und Bekanntmachung des Hinweises auf die Patenterteilung: **17.08.2022**
(21) Anmeldenummer: 16709371.5
(22) Anmeldetag: 09.03.2016
(51) Int. Cl.: A61B 17/80, A61B 17/17, A61B 34/10, A61B 17/56, A61B 17/00

(54) **ORTHOGNATISCHES SÄGE- UND POSITIONIERUNGSIMPLANTAT**
ORTHOGNATHIC SAW AND POSITIONING IMPLANT
IMPLANT ORTHOGNATHIQUE DE SCIAGE ET DE POSITIONNEMENT

(30) Priorität: 12.05.2015 DE 102015107484
(43) Veröffentlichungstag der Anmeldung: 21.03.2018
(73) Patentinhaber: Karl Leibinger Medizintechnik GmbH & Co. KG, 78570 Mühlheim (DE)
(72) Erfinder: WAIZENEGGER, Axel, 78570 Mühlheim (DE)
(74) Vertreter: Winter, Brandl - Partnerschaft mbB
(86) Internationale Anmeldenummer: PCT/EP2016/054988
(87) Internationale Veröffentlichungsnummer: WO 2016/180557

(56) Entgegenhaltungen:
- WO-A1-2014/043370
- WO-A2-2014/090964
- DE-U1- 29 909 025
- US-A- 5 690 631
- US-A1- 2012 022 604

## Beschreibung

Die Erfindung betrifft ein orthognatisches Knochenverbindungsimplantat (auch als Positionierungsimplantat bezeichnet) für eine Osteotomieanwendung / zum Verbinden eines ersten Knochenbereiches mit einem zweiten Knochenbereich oder mehreren Knochenbereichen eines Säugerknochens / Knochens eines Säugetieres, mit einem mehrere Befestigungsmittelaufnahmelöcher aufweisenden, zur Anbringung an dem ersten Knochenbereich vorbereiteten ersten Befestigungsbereich und einem mit dem ersten Befestigungsbereich verbundenen, zweiten Befestigungsbereich, wobei der zweite Befestigungsbereich wiederum mehrere Befestigungsmittelaufnahmelöcher aufweist und zur Anbringung an dem zweiten Knochenbereich vorbereitet ist. Gerade wenn etwa der Unterkiefer zweiteilig ist, bspw. bei einem Unterkieferengstand, gibt es mehrere Knochenbereiche mit denen der erste Knochenbereich verbunden wird. Unter einem Säugerknochen / einem Knochen eines Säugetieres wird ein besonders hartes, skelettbildendes Stützgewebe eines Wirbellebewesens verstanden, also eine solche Struktur aus Knochengewebe. Insbesondere sind dadurch solche Knochen, wie Fibula und Tibea aber auch Schädelknochen mit umfasst.

Aus dem Stand der Technik sind gattungsgemäße Knochenverbindungsimplantate bereits bekannt. In diesem Zusammenhang offenbart beispielsweise die WO 2014/090964 A2 ein Implantat sowie eine Führung, zusammen mit Verfahren zum Konfigurieren derselben. Das Implantat sowie die Führung sind für Osteotomieanwendungen an einer Maxilla eines Patienten vorgesehen und können als Bausatz ausgestaltet sein. Die dreidimensionalen Modelle der prä- und postoperativen Anatomie werden dabei verwendet um die Befestigungsbereiche für die Führung sowie das Implantat zu definieren. Diese Befestigungsbereiche sind dann weiter dafür genutzt, um die Struktur des Implantates sowie die Führung zu definieren. Weiterer Stand der Technik ist aus der EP 2 698 122 A1, der WO 2011/136898 A1, der WO 2013/156545 A1 und der US 2007/0276383 A1 bekannt.

Auch ist aus der WO 2014/043370 A1 ist ein Knochenimplantat zur Verkürzung eines Knochens bekannt, bei dem eine Platte derart auf einem Knochen positioniert ist, dass ein mehrere Befestigungsmittelaufnahmelöcher aufweisender erster Abschnitt der Platte und ein mehrere Befestigungsmittelaufnahmelöcher aufweisender zweiter Abschnitt der Platte zur Anbringung an unterschiedlichen Knochenbereichen vorbereitet sind. Eine U-förmige Schnittführung erfüllt hierbei die Funktion, ein Herausschneiden eines Knochenabschnitt zu führen.

Zudem offenbart die US 5 690 631 A offenbart ein Osteosynthese-Implantat mit einer einstellbaren Platte, die von Verbindungselementen definierte Leerräumen sowie Befestigungsabschnitte zur Anbringung an einem Knochen mittels Schrauben besitzt.

Ferner offenbart die DE 299 09 025 U1 ein Implantat, das zur Kompressions-Osteosynthese dient und eine Knochenschraube aufweist, die derart in ein Kompressionsloch einschraubbar, dass sie aufgrund der Form von Ansenkungen mit zunehmender Verschraubungstiefe eine Kompression der Knochen hervorruft.

Sowohl die Druckschrift WO 2014/090964 A2, als auch die Druckschrift EP 2 698 122 A1 zeigen unterschiedliche Implantate, von denen jeweils ein Implantat vor dem Durchführen einer Osteotomie eingesetzt ist und ein davon anderes Implantat zum Verbinden der voneinander getrennten Knochenbereiche eingesetzt wird.

Diese aus dem Stand der Technik bekannten Ausführungen haben jedoch zumeist den Nachteil, dass für eine trennende Bearbeitung des jeweiligen, zu korrigierenden und anschließend wieder zu verbindenden Säugerknochens, beispielsweise einer Maxilla oder Mandibel, zwei separate Elemente verwendet werden müssen. Dabei wird die Durchtrennung des Säugerknochens, vorzugsweise mittels eines Sägeverfahrens, mit einer schablonenartigen Werkzeugführung umgesetzt und die Verbindung der beiden zuvor getrennten Knochenbereiche in der gewünschten Stellung mittels eines Implantates umgesetzt. Dadurch war es bisher für Osteotomieanwendungen stets notwendig, sowohl eine benutzerdefinierte Werkzeugführung, als auch ein benutzerdefiniertes Positionierungsimplantat herzustellen. Dies bedingte ein relativ aufwändiges Herstellen der für die Osteotomieanwendungen verwendeten Elemente und dadurch indirekt auch relativ hohe Operationskosten.

Es ist daher die Aufgabe der vorliegenden Erfindung, diese aus dem Stand der Technik bekannten Nachteile zu beheben und insbesondere ein Knochenverbindungsimplantat zur Verfügung zu stellen, das eine Osteotomiebehandlung in ihrem Aufwand weiter reduziert, wobei gleichzeitig eine patientenspezifische / individualisierte Anpassung des Knochenverbindungsimplantat gewahrt bleiben soll.

Die Aufgabe der Erfindung wird durch ein Knochenimplantat mit den Merkmalen des Patentanspruchs 1 gelöst.

Die Aufgabe der Erfindung wird bei einem Knochenimplantat erfindungsgemäß dadurch gelöst, dass zwischen dem ersten und dem zweiten Befestigungsbereich eine, eine (erste) Durchtrennungslinie vorgebende (erste) Trennwerkzeugführungskontur ausgebildet ist. Mit einer Lage zwischen dem ersten und dem zweiten Befestigungsbereich wird hierbei jene (räumliche) Lage bezeichnet, die sich, in einer räumlichen Ersteckung entlang des Säugerknochens gesehen, zwischen dem ersten und dem zweiten Befestigungsbereich befindet. Die Trennwerkzeugführungskontur ist damit zwischen einer dem zweiten Befestigungsbereich zugewandten Seite des ersten Befestigungsbereiches sowie einer dem ersten Befestigungsbereich zugewandten Seite des zweiten Befestigungsbereiches angeordnet.

Durch eine solche Ausbildung ist es möglich, das Knochenverbindungsimplantat nicht nur als Positionierungsimplantat, sondern auch für die vorherige Trennung der beiden Knochenbereiche des Säugerknochens als Trennschablone zu verwenden. Dadurch wird die Korrektur der jeweiligen Fehlbildung des Säugerknochens besonders kostengünstig umgesetzt. Als weiterer Vorteil unter Verwendung ein und desselben Implantates für die Durchtrennung und die erneute Verbindung ist insbesondere auch zu nennen, dass Fertigungstoleranzen zwischen der bisher separat ausgeführten Werkzeugführung und dem Positionierungsimplantat vermieden werden. Die beiden Knochenbereiche sind dann nach dem Verbinden deutlich präziser in der zuvor errechneten Sollstellung zueinander angeordnet, sodass der Heilungsprozess des Säugerknochens weiter gefördert wird.

Die Trennwerkzeugführungskontur ist durch eine Innenkante einer zwischen dem ersten und dem zweiten Befestigungsbereich ausgestalteten / ausgebildeten Rahmenstruktur ausgebildet / ausgeformt, so dass die Trennwerkzeugführungskontur an einem besonders formstabilen Bereich des Knochenverbindungsimplantats angeordnet ist und dessen Struktur nicht einfach durch die Trennoperation verändert werden kann.

Zudem weist das Knochenimplantat eine zwischen dem zweiten Befestigungsbereich und einem weiteren dritten Befestigungsbereich angeordnete, eine (zweite) Durchtrennungslinie vorgebende weitere / zweite Trennwerkzeugführungskontur auf, wobei der dritte Befestigungsbereich wiederum mehrere Befestigungsmittelaufnahmelöcher aufweist und zur Anbringung an dem ersten Knochenbereich vorbereitet ist. Dadurch werden die beiden Knochenbereiche in einem mit beiden Knochenbereichen befestigten Zustand noch stabiler relativ zueinander lagegesichert. Die zweite Trennwerkzeugführungskontur ist dabei weiter bevorzugt wie die erste Trennwerkzeugführungskontur ausgebildet.

Weitere vorteilhafte Ausführungsformen sind in den Unteransprüchen beansprucht und nachfolgend näher erläutert.

Gemäß einer bevorzugten Ausführungsform kann die Trennwerkzeugführungskontur an einem dem ersten Befestigungsbereich zugeordneten Verbindungssteg ausgebildet sein, wobei eine Unterseite des Verbindungsstegs wellenförmig ausgebildet ist. Dies hat den Vorteil, dass eine Anlagefläche für das zum Knochentrennen eingesetzten Werkzeug reduziert werden kann, so dass eine Kontaktfläche zu dem Trennwerkzeug, wie einem Sägeblatt, minimiert wird.

Vorteilhaft ist es im Weiteren, wenn die (erste) Trennwerkzeugführungskontur leistenförmig ausgestaltet ist. Dadurch ist das Durchtrennungswerkzeug, etwa eine Säge / Kreissäge, an der Trennwerkzeugführungskontur besonders stabil abgestützt, um die beiden Knochenbereiche entlang der Durchtrennungslinie / Osteotomielinie zu durchtrennen.

Zudem ist es von Vorteil, wenn die Trennwerkzeugführungskontur unmittelbar durch einen mit dem ersten Befestigungsbereich und/oder dem zweiten Befestigungsbereich verbundenen Verbindungssteg ausgebildet ist. Dadurch ist das Knochenverbindungsimplantat besonders kompakt aufgebaut.

Wenn die (ersten und zweiten) Befestigungsbereiche und die Trennwerkzeugführungskontur stoffeinteilig / integral miteinander ausgebildet / verbunden sind, ist das Knochenverbindungsimplantat noch stabiler. Die Befestigungsbereiche und die Trennwerkzeugführungskontur sind dabei weiter bevorzugt formstabil zueinander ausgebildet. Auch ist es von Vorteil, wenn das Knochenverbindungsimplantat aus einem biokompatiblen und/oder bioresorbierbaren Werkstoff hergestellt ist / vollständig besteht. Dadurch ist das Knochenverbindungsimplantat besonders effizient einsetzbar.

In diesem Zusammenhang ist es auch besonders vorteilhaft, wenn das Knochenverbindungsimplantat aus einem Metallwerkstoff, vorzugsweise einem Titanwerkstoff, hergestellt ist. Der Titanwerkstoff ist weiter bevorzugt wärmebehandelt. Somit ist ein in sich besonders formstabiles Knochenverbindungsimplantat umgesetzt.

Vorteilhaft ist es auch, wenn das Knochenverbindungsimplantat zum Verbinden eines ersten Knochenbereiches mit einem zweiten Knochenbereich eines Oberkieferknochens / einer Maxilla oder eines Unterkieferknochens / eines Mandibel vorbereitet ist. Dadurch ist das Knochenverbindungsimplantat besonders effektiv wirkend.

Im Weiteren betrifft die Erfindung auch ein Verfahren zum individualisierten Herstellen des Knochenimplantats nach zumindest einem der zuvor ausgeführten Ausführungsformen, umfassend folgende, vorzugsweise zeitlich nacheinander ablaufende Schritte:
a) Erfassen eines 3D-Ist-Modelles des zu behandelnden Säugerknochens in einem ersten Datensatz,
b) Erstellen eines 3D-Soll-Modelles in einem zweiten Datensatz unter Festlegen zwei Durchtrennungslinien an dem 3D Ist-Modell sowie unter einem relativen Verschieben zweier imaginärer Knochenbereiche (nämlich des ersten Knochenbereiches relativ zum zweiten Knochenbereich) zueinander, und
c) Fertigen des Knochenverbindungsimplantats anhand des 3D Soll-Modelles / des zweiten Datensatzes, wobei der erste Befestigungsbereich zur Befestigung an dem ersten (imaginären) Knochenbereich des 3D Soll-Modelles, der zweite Befestigungsbereich zur Befestigung an dem zweiten (imaginären) Knochenbereich des 3D Soll-Modelles, der dritte Befestigungsbereich zur Befestigung an dem ersten Knochenbereich und die Trennwerkzeugkonturen unter einem zumindest teilweisen Nachbilden der Durchtrennungslinie ausgeformt werden. Dadurch ist ein besonders effektives Herstellen eines Knochenverbindungsimplantats umgesetzt.

Im Weiteren wird auch ein Verfahren zum Behandeln eines vorzugsweise menschlichen Säugerknochens unter Verwendung eines Knochenverbindungsimplantats nach einem der zuvor genannten Ausführungsformen beschrieben. Bei diesem Verfahren handelt es sich nicht um einen Teil der Erfindung. Es dient lediglich dem Verständnis der Erfindung. Das Verfahren umfasst folgende Schritte:
a) Anbringen des Knochenverbindungsimplantates mit einem ersten Befestigungsbereich an einem ersten Knochenbereich des Säugerknochens,
b) Durchtrennen des Säugerknochens entlang einer Durchtrennungslinie unter Anlage eines Trennwerkzeuges an der Trennwerkzeugführungskontur,
c) Ausrichten des von dem ersten Knochenbereich abgetrennten zweiten Knochenbereichs in der gewünschten Sollstellung, und
d) Befestigen des zweiten Befestigungsbereiches an dem zweiten Knochenbereich.

Dadurch ist auch ein Behandlungsverfahren besonders effektiv ausgebildet.

Die Erfindung wird nachfolgend anhand von Figuren näher erläutert.

Es zeigen:

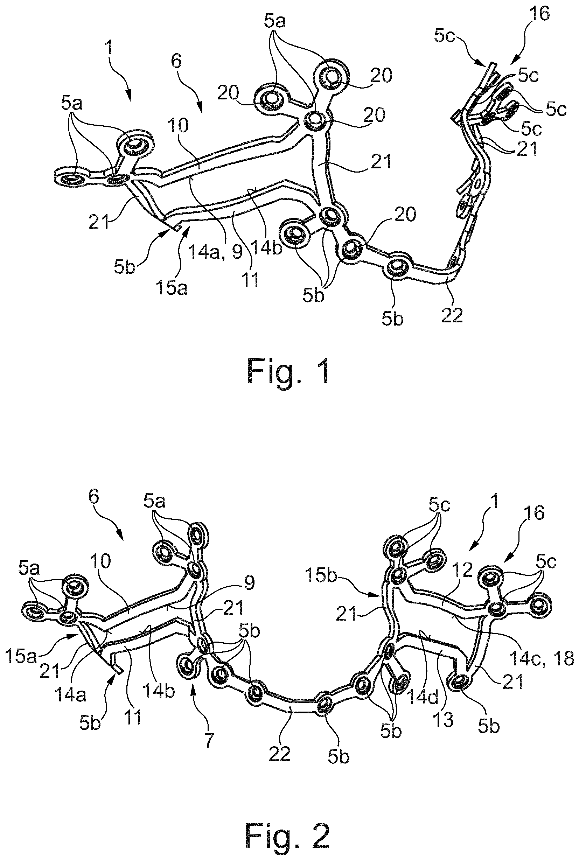

- Fig. 1: eine isometrische Darstellung eines erfindungsgemäßen Knochenverbin-dungsimplantats nach einem vorteilhaften Ausführungsbeispiel, wobei besonders gut ein erster und ein zweiter Befestigungsbereich sowie eine an einem Verbindungssteg des ersten Befestigungsbereiches ausgebildete (erste) Trennwerkzeugführungskontur ersichtlich ist,
- Fig. 2: eine Vorderansicht des in Figur 1 dargestellten Knochenverbindungsimplantats, wobei nun neben dem ersten und dem zweiten Befestigungsbereich ein weiterer, mit dem zweiten Befestigungsbereich ebenfalls verbundener, dritter Befestigungsbereich zu erkennen ist, welcher dritte Befestigungsbereich wiederum einen eine (zweite) Trennwerkzeugführungskontur ausbildenden Verbindungssteg aufweist,

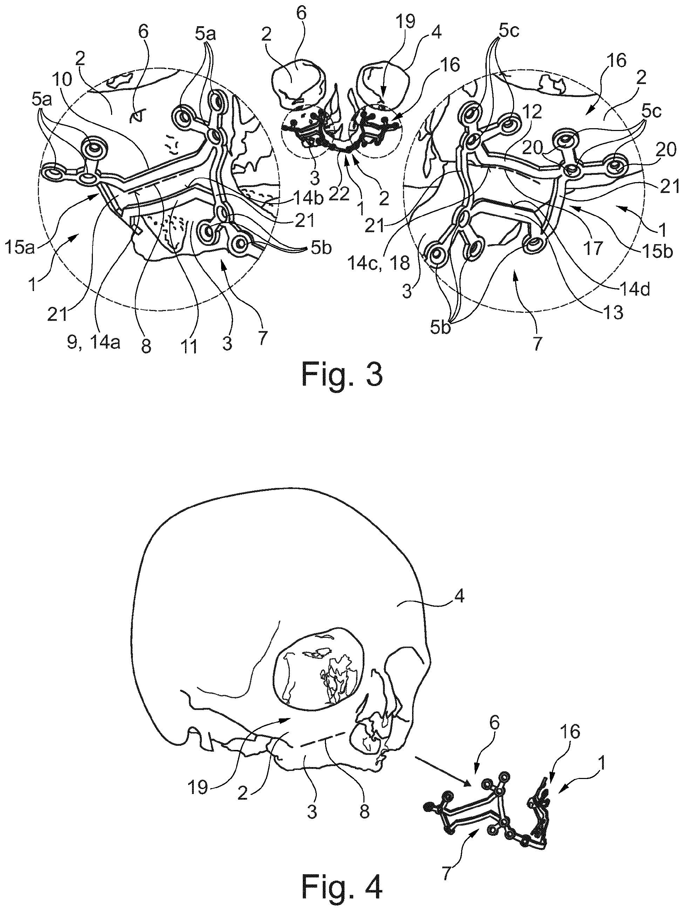

- Fig. 3: eine Vorderansicht eines 3D-Ist-Modelles eines als menschlicher Schädel ausgebildeten Säugerknochens, wobei gemäß den beiden Detailnebendarstellungen der erste und der dritte Befestigungsbereich des Knochenverbindungsimplantates nach den Fign. 1 und 2 jeweils an einer Maxilla des Schädels befestigt sind und die Durchtrennungslinien an den Trennwerkzeugführungskonturen optisch hervorgehoben sind,
- Fig. 4: eine isometrische Darstellung des in Fig. 3 gezeigten 3D-Ist-Modelles nach Durchführung eines mittels eines Trennwerkzeuges ausgebildeten Teilschnittes in der Maxilla entlang der Trennwerkzeugführungskonturen sowie nach anschließender Abnahme des Knochenverbindungsimplantats von dem 3D-Ist-Modell,

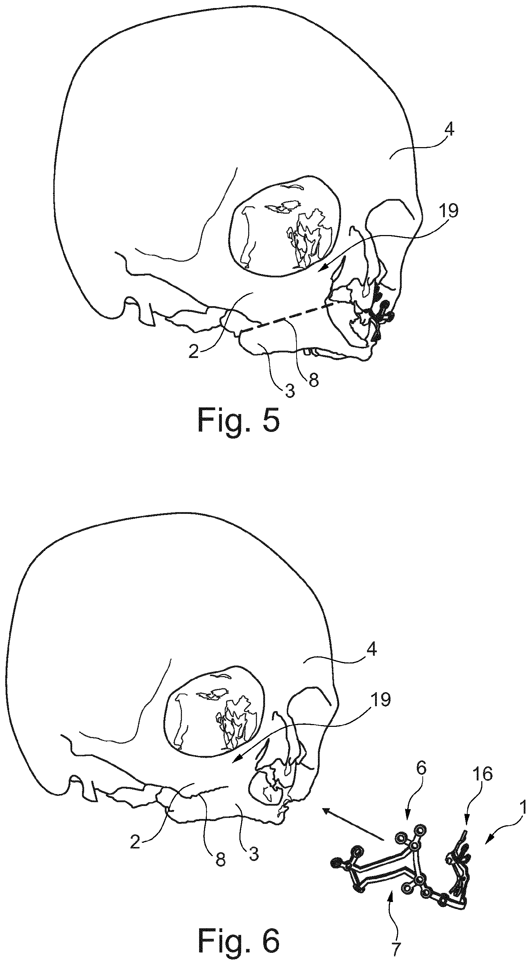

- Fig. 5: eine isometrische Darstellung des 3D-Ist-Modelles nach Durchführung einer vollständigen Durchtrennung der Maxilla entlang der zuvor teilweise ausgebildeten Durchtrennungslinien, wobei eine Durchtrennungslinie optisch hervorgehoben ist,
- Fig. 6: eine isometrische Darstellung des 3D-Ist-Modelles mit denn beiden zuvor getrennten Knochenbereichen der Maxilla vor dem erneuten Befestigen des Knochenverbindungsimplantats,

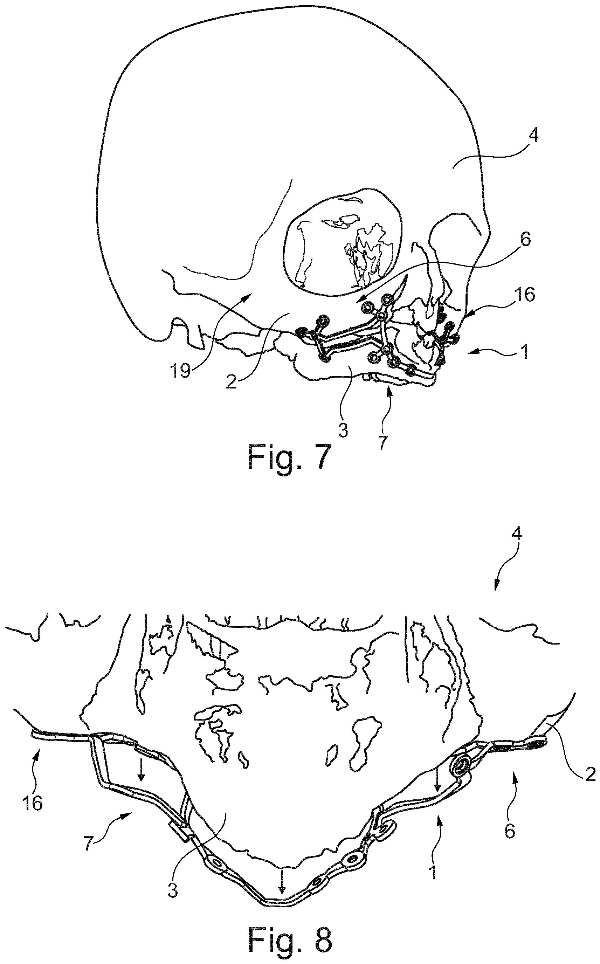

- Fig. 7: eine isometrische Darstellung des 3D-Ist-Modelles in einem wieder aufgesetzten Zustand des Knochenverbindungsimplantats, in dem die ersten und dritten Befestigungsbereiche wiederum an dem ersten Knochenbereich angebracht sind, der zweite Befestigungsbereich jedoch noch beabstandet zu dem zweiten Knochenbereich ist,
- Fig. 8: eine Unteransicht des 3D-Ist-Modelles, in der der Schädel von einer Unterseite dargestellt ist, in der der zweite Knochenbereich relativ zu dem ersten Knochenbereich entlang der Verschiebepfeile so lange verschoben wird bis der zweite Knochenbereich an dem zweiten Befestigungsbereich anliegt,

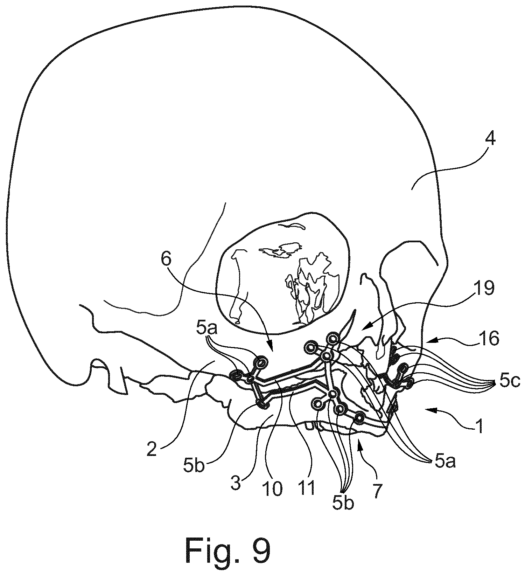

- Fig. 9: eine isometrische Darstellung des 3D-Ist-Modelles mit einem vollständig angebrachten Knochenverbindungsimplantat, das sowohl in den beiden ersten und dritten als auch in dem zweiten Befestigungsbereich fest mit dem ersten bzw. dem zweiten Knochenbereich verbunden ist, und

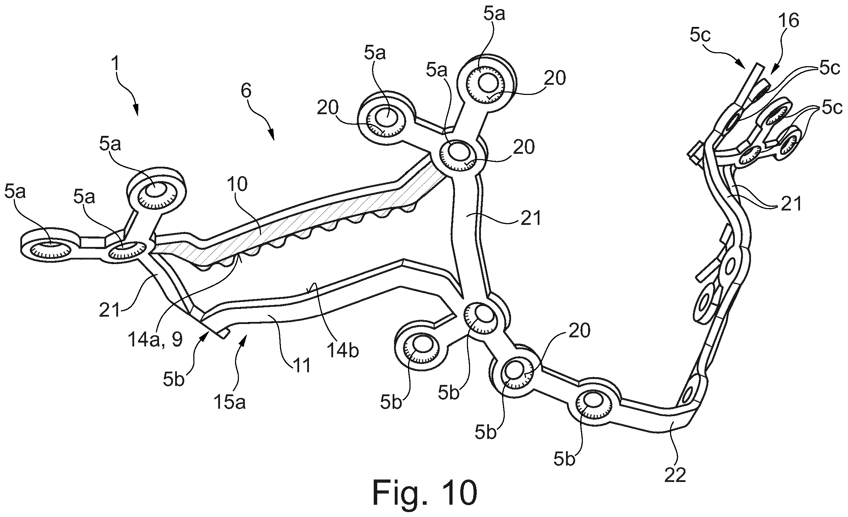

- Fig. 10: eine weitere Ausführungsform, in der eine Unterseite eines oberen Verbindungssteges wellenförmig oder zickzackförmig ausgebildet ist.

Die Figuren sind lediglich schematischer Natur und dienen ausschließlich dem Verständnis der Erfindung. Die gleichen Elemente sind mit denselben Bezugszeichen versehen.

In Figur 1 ist besonders gut ein erfindungsgemäßes Knochenverbindungsimplantat 1 nach einem bevorzugten Ausführungsbeispiel zu erkennen. In dieser Abbildung ist insbesondere ein mehrere Befestigungsmittelaufnahmelöcher 5a aufweisender, zur Anbringung an einem ersten Knochenbereich 2 eines Säugerknochens 4 vorbereiteter erster Befestigungsbereich 6 sowie ein mit dem ersten Befestigungsbereich 6 verbundener zweiter Befestigungsbereich 7 zu erkennen. Auch der zweite Befestigungsbereich 7 weist wiederum mehrere Befestigungsmittelaufnahmelöcher, nachfolgend als zweite Befestigungsmittelaufnahmelöcher 5b bezeichnet, auf, wodurch der zweite Befestigungsbereich 7 zur Anbringung an einem zweiten Knochenbereich 3 des Säugerknochens 4 vorbereitet ist.

Wie dann weiterhin in Figur 2 gut zu erkennen ist, ist der zweite Befestigungsbereich 7, der im Wesentlichen mittels eines Hauptsteges 22 gebildet ist auch mit einem dritten Befestigungsbereich 16 verbunden. An dem Hauptsteg 22 sind die zweiten Befestigungsmittelaufnahmelöcher 5b kettenartig nebeneinander angeordnet. Der Hauptsteg 22 bildet wiederum, wie nachfolgend näher beschrieben einen zweiten und einen dritten Verbindungssteg 11, 13 aus. Der dritte Befestigungsbereich 16 ist dabei wiederum im Wesentlichen wie der erste Befestigungsbereich 6 ausgebildet und weist ebenfalls mehrere Befestigungsmittelaufnahmelöcher, die nachfolgend als dritte Befestigungsmittelaufnahmelöcher 5c bezeichnet sind, auf. Diese dritten Befestigungsmittelaufnahmelöcher 5c dienen, wie nachfolgend näher erläutert, wiederum zur Befestigung an dem ersten Knochenbereich 2. Sowohl der erste Befestigungsbereich 6 als auch der dritte Befestigungsbereich 16 weisen jeweils zwei Gruppen dreieckförmig zueinander angeordneter erster bzw. dritter Befestigungsmittelaufnahmelöcher 5a, 5c auf.

Die ersten Befestigungsmittelaufnahmelöcher 5a sind an einem dem ersten Befestigungsbereich 6 zugeordneten, ersten Verbindungssteg 10 angeordnet, der in einem an dem Säugerknochen 4 befestigten Zustand im Wesentlichen horizontal ausgerichtet ist. Somit bildet der erste Befestigungsbereich 6 den ersten Verbindungssteg 10 aus, der leistenförmig ausgebildet ist und die beiden Gruppen der ersten Befestigungsmittelaufnahmelöcher 5a (mit jeweils drei ersten Befestigungsmittelaufnahmelöchern 5a) miteinander verbindet. Mit dem ersten Befestigungsbereich 6 sind dann wiederum zwei im Wesentlichen senkrecht zu dem ersten Verbindungssteg 10 ausgerichtete Überbrückungsstege 21 verbunden. Jeder der Überbrückungsstege 21 ist an den ersten Befestigungsbereich 6 im Bereich eines Befestigungsmittelaufnahmeloches 5a angeformt. Die Überbrückungsstege 21 verbinden den ersten Befestigungsbereich 6 bzw. den ersten Verbindungssteg 10 mit dem an dem zweiten Befestigungsbereich 7 ausgebildeten, ebenfalls leistenförmigen, zweiten Verbindungssteg 11, der sich im Wesentlichen parallel zu dem ersten Verbindungssteg 10 erstreckt. Die beiden Überbrückungsstege 21 sowie der zweite Verbindungssteg 11 des zweiten Befestigungsbereiches 7 bilden zusammen mit dem ersten Verbindungssteg 10 des ersten Befestigungsbereiches 6 eine erste im Wesentlichen rautenförmige / rechteckförmige Rahmenstruktur 15a aus.

Auf gleiche Weise ist dann der dritte Befestigungsbereich 16 mit dem zweiten Befestigungsbereich 7 verbunden. Die dritten Befestigungsmittelaufnahmelöcher 5c sind an einem dem dritten Befestigungsbereich 16 zugeordneten, dritten Verbindungssteg 12 angeordnet, der in einem an dem Säugerknochen 4 befestigten Zustand im Wesentlichen horizontal ausgerichtet ist. Somit bildet der dritte Befestigungsbereich 16 den dritten Verbindungssteg 12 aus, der leistenförmig ausgebildet ist und die beiden Gruppen der dritten Befestigungsmittelaufnahmelöcher 5c (mit jeweils drei dritten Befestigungsmittelaufnahmelöchern 5c) miteinander verbindet. Mit dem dritten Befestigungsbereich 16 sind dann wiederum zwei im Wesentlichen senkrecht zu dem dritten Verbindungssteg 12 ausgerichtete Überbrückungsstege 21 verbunden. Jeder der Überbrückungsstege 21 ist an den dritten Befestigungsbereich 16 im Bereich eines Befestigungsmittelaufnahmeloches 5c angeformt. Die Überbrückungsstege 21 verbinden den dritten Befestigungsbereich 16 bzw. den dritten Verbindungssteg 12 mit einem an dem zweiten Befestigungsbereich 7 ausgebildeten, ebenfalls leistenförmigen, vierten Verbindungssteg 13, der sich im Wesentlichen parallel zu dem dritten Verbindungssteg 12 erstreckt. Die beiden Überbrückungsstege 21 sowie der vierte Verbindungssteg 13 des zweiten Befestigungsbereiches 7 bilden zusammen mit dem dritten Verbindungssteg 12 des dritten Befestigungsbereiches 16 eine zweite im Wesentlichen rautenförmige / rechteckförmige Rahmenstruktur 15b aus.

In dieser Ausführung ist die erste Rahmenstruktur 15a etwas anders ausgestaltet als die zweite Rahmenstruktur 15b. Die zweite Rahmenstruktur 15b ist hierbei derart anders ausgebildet, dass sich ein Abstand zwischen dem dritten und vierten Verbindungssteg 12, 13 größer ist als ein Abstand zwischen dem ersten und dem zweiten Verbindungssteg 10, 11.

Die dritten Befestigungsmittelaufnahmelöcher 5c sind wiederum gleich wie die ersten sowie die zweiten Befestigungsmittelaufnahmelöcher 5a, 5b ausgebildet. Die Befestigungsmittelaufnahmelöcher 5a, 5b, 5c bilden allesamt auf übliche Weise Aufnahmen für Befestigungsmittel in Form von Knochenschrauben aus, wobei jedes der Befestigungsmittelaufnahmelöcher 5a, 5b, 5c an einer dem jeweiligen Knochenbereich 2, 3 abgewandten Seite eine konische Schraubenkopfanlagefläche 20 aufweist. Im befestigten Zustand des Knochenverbindungsimplantats 1 an den beiden Knochenbereichen 2, 3 sind dann die Schraubenköpfe der Knochenschrauben vollständig in diesen Befestigungsmittelaufnahmelöchern 5a, 5b, 5c versenkt.

Wie unter Zusammenwirkung der Figuren 1 und 2 weiterhin gut zu erkennen ist, sind sowohl die Befestigungsbereiche 6, 7, 16 als auch jeweils eine an der jeweiligen Rahmenstruktur 15a, 15b angeordnete Trennwerkzeugführungskontur 9, 18 integral, d. h. stoffeinteilig, miteinander ausgebildet.

In dieser Ausführung bildet eine Innenkante, nämlich eine erste Innenkante 14a des ersten Verbindungssteges 10 unmittelbar eine erste Trennwerkzeugführungskontur 9 aus, die vorbereitet ist, als Führungsschiene für ein Trennwerkzeug, nämlich ein Sägewerkzeug / eine Kreissäge, zu dienen. Die erste Trennwerkzeugführungskontur 9 bildet eine anzufertigende erste Durchtrennungslinie 8 in dem Säugerknochen 4 nach. Alternativ oder zusätzlich hierzu ist es auch möglich, die (zweite) Innenkante 14b des zweiten Verbindungssteges 11 als eine solche erste Trennwerkzeugführungskontur 9 auszugestalten. Die erste und die zweite Innenkante 14a, 14b sind jene Seitenkanten der Verbindungsstege 10, 11, die einander zugewandt sind.

Im Weiteren ist auch die (dritte) Innenkante 14c des dritten Verbindungssteges 12 als Trennwerkzeugführungskontur, nämlich als zweite Trennwerkzeugführungskontur 18 ausgebildet. Auch die zweite Trennwerkzeugführungskontur 18 dient hierbei als Führungsschiene für ein Trennwerkzeug, nämlich ein Sägewerkzeug / eine Kreissäge beim Trennen des ersten Knochenbereiches 2 von dem zweiten Knochenbereich 3. Die zweite Trennwerkzeugführungskontur 18 bildet eine anzufertigende zweite Durchtrennungslinie 17 in dem Säugerknochen 4 nach. Alternativ oder zusätzlich hierzu ist es auch möglich, wiederum die (vierte) Innenkante 14d des vierten Verbindungssteges 13 als eine solche zweite Trennwerkzeugführungskontur 18 vorzusehen. Die dritte und die vierte Innenkante 14c, 14d sind jene Seitenkanten der Verbindungsstege 10, 11, die einander zugewandt sind.

Der zweite und der vierte Verbindungssteg 10, 11 sind auch integraler Bestandteil des Hauptsteges 22, der die beiden flügelartig angeordneten Rahmenstrukturen 15a, 15b miteinander formstabil verbindet. Auch sei darauf hingewiesen, es gemäß einer weiteren Ausführungsform umgesetzt ist, dass der Hauptsteg 22 mittig zwischen den Rahmenstrukturen 15a, 15b mit einem wiederverschließbaren Mechanismus ausgestaltet ist, woraufhin die Rahmenstrukturen 15a, 15b unabhängig voneinander an den Knochenbereichen 2, 3 befestigbar sind und im Anschluss daran wieder über den Mechanismus formstabil miteinander verbunden werden können.

Das Knochenverbindungsimplantat 1 ist durch seine Ausbildung als Implantat aus einem biokompatiblen Werkstoff, nämlich einem gehärteten Titanwerkstoff ausgebildet / hergestellt. Das Knochenverbindungsimplantat 1 kann zusätzlich oder alternativ dazu auch aus einem bioresorbierbaren Werkstoff / bioresorbierbar teilweise oder vollständig hergestellt sein.

In Verbindung mit den Figuren 3 bis 9 ist dann besonders gut auch ein Verfahren zum Herstellen eines erfindungsgemäßen Knochenimplantats 1 zu erkennen. Hierfür wird zunächst, wie beispielsweise in Figur 3 erkennbar, ein 3D-Ist-Modell des hier als menschlichen Schädel ausgeführten, mittels Osteotomie zu behandelnden Säugerknochens 4 erstellt. Dies erfolgt mittels einer tomografischen Bilderfassungseinrichtung (CT-Verfahren), die den Säugerknochens 4 abscannt und einen ersten Datensatz ermittelt, der die dreidimensionale Form des Säugerknochens 4 enthält / wiederspiegelt.

Dieser Säugerknochen 4 weist bereits eine Fehlverformung einer Maxilla / eines Oberkieferknochens 19 des Säugerknochens 4 auf, die durch eine trennende Dysgnathie-Chirurgie / eine Osteotomiebehandlung zu beheben ist. Anhand dieses imaginären 3D-Ist-Modelles wird dann anschließend ein 3D-Soll-Modell der Maxilla / des Säugerknochens 4 erstellt, wobei je erstem und drittem Befestigungsbereich 6, 16, jeweils eine Durchtrennungslinie 8 bzw. 17 an dem imaginären 3D-Ist-Modell festgelegt wird. Den Durchtrennungslinien 8, 17, die an dem 3D-Ist-Modell angeordnet werden, wird dann jeweils eine der Trennwerkzeugführungskonturen 9, 18 zugeordnet bzw. wird dann jeweils eine der Trennwerkzeugführungskonturen 9, 18 entsprechend dieser Durchtrennungslinien 8, 17 angeformt. Nach dem Festlegen dieser beiden Durchtrennungslinien 8, 17 werden die beiden Knochenbereiche 2, 3 imaginär voneinander getrennt und relativ zueinander in die gewünschte Relativposition verschoben, sodass sich schließlich ein imaginäres 3D-Soll-Modell (in einem zweiten Datensatz errechnet), in Fig. 9 ergibt, wonach die Befestigungsbereiche 6, 7, 16 an die Form dieses 3D-Soll-Modelles angepasst werden. Die Befestigungsbereiche 6, 7, 16 werden so zueinander angeglichen und verformt, dass die ersten und dritten Befestigungsbereiche 6, 16 zur flächigen Anlage an dem ersten Knochenbereich 2 angepasst werden und der zweite Befestigungsbereich 7 zur flächigen Anlage an dem zweiten Knochenbereich 3 angepasst wird.

Auch ist in Verbindung mit den Figuren 3 bis 9 besonders gut ein Behandlungsverfahren des Säugerknochens 4 / Schädels zu erkennen. Hierfür wird zunächst das bereits angefertigte Knochenverbindungsimplantat 1 mittels der ersten und der dritten Befestigungsbereiche 6, 16 an dem Säugerknochen 4 befestigt, während der zweite Befestigungsbereich 7 noch getrennt von dem zweiten Knochenbereich 3 der Maxilla bleibt (Fig. 3). Im Anschluss daran werden dann entlang der durch die Trennwerkzeugführungskonturen 9 und 18 gebildeten Durchtrennungslinien 8, 17 die beiden Knochenbereiche 2, 3 partiell innerhalb der Rahmenstrukturen 15a und 15b voneinander getrennt, was durch die Detailnebendarstellungen in Fig. 1 zu erkennen ist. Im Anschluss daran wird dann gemäß Figur 4 das Knochenverbindungsimplantat 1 von dem ersten Knochenbereich 2 gelöst und abgenommen und der erste Knochenbereich 2 in dem Schritt nach Figur 5 vollständig von dem zweiten Knochenbereich 3 durch weiteres Sägen entlang der bereits vorgezeichneten Durchtrennungslinie 8, 17 abgetrennt. Nachdem die beiden Knochenbereiche 2 und 3 vollständig voneinander abgetrennt sind, wird das Knochenverbindungsimplantat 1 wiederum mit dem ersten 6 sowie dem dritten Befestigungsbereich 16 an dem ersten Knochenbereich 2 befestigt (Fig. 6), wobei in jedem Befestigungsmittelaufnahmeloch 5a, 5c jeweils eine Knochenschraube eingebracht wird, die wiederum in den ersten Knochenbereich 2 eingeschraubt wird. In einem befestigten Zustand der beiden Befestigungsbereiche 6 und 16 an dem ersten Knochenbereich 2 nach Figur 7 wird dann gemäß Figur 8 der, von dem ersten Knochenbereich losgelöste, zweite Knochenbereich 3 relativ zu dem ersten Knochenbereich 2 verschoben, bis gemäß Figur 9 der zweite Knochenbereich 3 insbesondere im Bereich der zweiten Befestigungsmittelaufnahmelöcher 5b an dem zweiten Befestigungsbereich 7 anliegt. In dieser dann beabsichtigten korrigierten Stellung zwischen dem ersten und dem zweiten Knochenbereich 2, 3 werden dann wiederum mehrere Knochenschrauben in die zweiten Befestigungsmittelaufnahmelöcher 5b eingebracht und mit dem zweiten Knochenbereich 3 verschraubt. Dies führte letztendlich dazu, dass die beiden Knochenbereiche 2, 3 durch das Knochenverbindungsimplantat 1 fest miteinander verbunden sind.

In anderen Worten ausgedrückt, liegt der erfindungsgemäße Gedanke somit in der Vereinigung einer Sägeschablone und eines patientenspezifischen Orthognathie-Implantats in ein kombiniertes Säge- und Positionierungs-Implantat 1. Von Vorteil ist es dann insbesondere der, dass notwendige Positionierungshilfen wie z.B. Splinte, Navigationsgeräte, Markierungsschrauben und Fräßlinien wegfallen können. Auch die zusätzliche Bohrschablone fällt dann weg. Zudem wird die Präzision von Planungsumsetzung und operativen Eingriff verbessert, wobei auch die Keimbelastung durch Wegfall eines zusätzlichen potentiellen Keimträgers verbessert wird. Der operative Ablauf wird auch durch die Reduktion der operativen Einzelschritte erleichtert. Zudem wird die OP Zeit durch Wegfall zusätzlicher Instrumentenwechsel und diese Reduktion der Einzelschritte verkürzt. Dadurch ist letztendlich auch eine kostengünstigere Produktion durch Reduktion der Produktionsschritte umgesetzt.

In der erfindungsgemäßen Ausführung des Knochenverbindungsimplantats 1 befinden sich pro Seite jeweils zwei horizontal gerichtet verlaufende Stege 10, 11; 12, 13 im Bereich der rechten und linken Kieferhöhlenwand, die von der Crista zygomaticoalveolaris bis zur jeweiligen lateralen Seite des Foramen piriformis ziehen. Diese beiden Stege 10, 11; 12, 13 bilden durch den gebildeten Zwischenraum / Schlitz jeweils eine Führung, die einer Sägeschablone entspricht. Der Zwischenraum kann auch aparallel verlaufen, wenn eine knöcherne Resektion durchgeführt werden soll. In diesem Fall dient die untere Kante 14a; 14c des oberen Stegs und die obere Kante des unteren Stegs 14b; 14d als Führung bei der Osteotomie. Bei Bedarf können die Stege 10, 11; 12, 13 mit Bohrlöchern versehen werden, um weitere Fixierungsmöglichkeiten zu erhalten. Verbunden sind die horizontal gerichteten Stege 10, 11; 12, 13 mit vier vertikal gerichteten Stegen 21, die einen Verbund zwischen oberem und unterem Stegepaar 10, 11; 12, 13 darstellt. Die geplante Verschiebeinformation wird in diesem Bereich durch Biegungen verschlüsselt. Die beiden horizontal gerichteten Stege 10, 11; 12, 13 rechts und links sind im äußeren Bereich (lateral) mit jeweils einem vertikalen Steg 21 verbunden, um so ausreichende Stabilität in diesem Bereich zu erhalten. Diese können bei Bedarf Richtung Jochbeinkörper verlängert werden, um hier zusätzliche Fixationsmöglichkeiten mit Osteosyntheseschrauben (Knochenschrauben) zu erhalten. Paranasal bds. befinden sich vertikal gerichtete Stege mit Bohrlöchern zur weiteren Fixierung. Ein horizontal gerichteter Steg 10, 11; 12, 13 verbindet rechte und linke Seite unterhalb der Spina nasales. Die Verbindung kann im Bereich der Spina nasales auch in situ durch eine Verankerung oder Schlossprinzip während der Operation durchgeführt werden, so dass bei initialem Einsetzen ein großes Implantat in Einzelteile zerlegt werden kann. Mehrere Kieferteile, wie z.B. die dreigeteilte LeFort I Osteotomie, können ebenfalls mit diesem Implantattyp versorgt werden.

In der Figur 10 ist zwar nur eine wellenförmige Unterseite eines Verbindungssteges 10 dargestellt, doch können mehrere solcher geometrisch abwechselnden Strukturen vorhanden sein, insbesondere an all den Stellen, die als Trennwerkzeugführungskontur 9 dienen. Dies hat den Vorteil, dass die Anlagefläche für das zum Knochentrennen eingesetzten Werkzeug, wie eines Sägeblattes, reduziert ist. Des Weiteren ist überlegt worden, dass es auch Sinn machen würde, wenn man den "Sägeführungsbereich" nach Einbringen des Sägeschnitts, abtrennen könnte, evtl. mittels Abzwickens, wie bei einem "Revell Bausatz", um die Materialeinbringung zu reduzieren. Dann könnte auch die Form wie ein Führungsschlitz ausgebildet werden, in dem z.B. ein oszillierendes Messer geführt wird. Die "Anschlagsleiste" kann zumindest einseitig "wellenförmig" ausgeprägt sein, eventuell auch auf zwei Seiten, um die Kontaktfläche zum Sägeblatt zu minimieren.

### Bezugszeichenliste

- 1: Knochenverbindungsimplantat
- 2: erster Knochenbereich
- 3: zweiter Knochenbereich
- 4: Säugerknochen
- 5a: erstes Befestigungsmittelaufnahmeloch
- 5b: zweites Befestigungsmittelaufnahmeloch
- 5c: drittes Befestigungsmittelaufnahmeloch
- 6: erster Befestigungsbereich
- 7: zweiter Befestigungsbereich
- 8: erste Durchtrennungslinie
- 9: erste Trennwerkzeugführungskontur
- 10: erster Verbindungssteg
- 11: zweiter Verbindungssteg
- 12: dritter Verbindungssteg
- 13: vierter Verbindungssteg
- 14a: erste Innenkante
- 14b: zweite Innenkante
- 14c: dritte Innenkante
- 14d: vierte Innenkante
- 15a: erste Rahmenstruktur
- 15b: zweite Rahmenstruktur
- 16: dritter Befestigungsbereich
- 17: zweite Durchtrennungslinie
- 18: zweite Trennwerkzeugführungskontur
- 19: Oberkieferknochen
- 20: Schraubenkopfanlagefläche
- 21: Überbrückungssteg
- 22: Hauptsteg

## Patentansprüche

1. Knochenverbindungsimplantat (1) zum Verbinden eines ersten Knochenbereiches (2) mit einem osteotomisch vom ersten Knochenbereich (2) getrennten zweiten Knochenbereich (3) oder mehreren Knochenbereichen eines Säugerknochens (4), mit einem mehrere Befestigungsmittelaufnahmelöcher (5a) aufweisenden, zur Anbringung an dem ersten Knochenbereich (2) vorbereiteten, ersten Befestigungsbereich (6) und einem mit dem ersten Befestigungsbereich (6) verbundenen, zweiten Befestigungsbereich (7), wobei der zweite Befestigungsbereich (7) wiederum mehrere Befestigungsmittelaufnahmelöcher (5b) aufweist und zur Anbringung an dem zweiten Knochenbereich (3) vorbereitet ist, wobei zwischen dem ersten und dem zweiten Befestigungsbereich (6, 7) eine eine Durchtrennungslinie (8) vorgebende Trennwerkzeugführungskontur (9) ausgebildet ist, wobei die Trennwerkzeugführungskontur (9) durch eine Innenkante (14a, 14b) einer zwischen dem ersten und dem zweiten Befestigungsbereich (6, 7) ausgestalteten Rahmenstruktur (15a) ausgebildet ist, **dadurch gekennzeichnet, dass** zwischen dem zweiten Befestigungsbereich (7) und einem dritten Befestigungsbereich (16) eine, eine Durchtrennungslinie (17) vorgebende, zweite Trennwerkzeugführungskontur (18) ausgebildet ist, wobei der dritte Befestigungsbereich (16) wiederum mehrere Befestigungsmittelaufnahmelöcher (5c) aufweist und zur Anbringung an dem ersten Knochenbereich (2) vorbereitet ist.

2. Knochenverbindungsimplantat (1) nach Anspruch 1, **dadurch gekennzeichnet, dass** die Trennwerkzeugführungskontur (9) an einem dem ersten Befestigungsbereich (6) zugeordneten Verbindungssteg (10) ausgebildet ist, wobei eine Unterseite des Verbindungsstegs (10) wellenförmig ausgebildet ist.

3. Knochenverbindungsimplantat (1) nach Anspruch 1 oder 2, **dadurch gekennzeichnet, dass** die Trennwerkzeugführungskontur (9) leistenförmig ausgestaltet ist.

4. Knochenverbindungsimplantat (1) nach einem der Ansprüche 1 bis 3, **dadurch gekennzeichnet, dass** die Trennwerkzeugführungskontur (9) unmittelbar durch einen mit dem ersten Befestigungsbereich (6) und/oder dem zweiten Befestigungsbereich (7) verbundenen Verbindungssteg (10, 11) ausgebildet ist.

5. Knochenverbindungsimplantat (1) nach einem der Ansprüche 1 bis 4, **dadurch gekennzeichnet, dass** die Befestigungsbereiche (6, 7) und die Trennwerkzeugführungskontur (9) stoffeinteilig ausgebildet sind.

6. Knochenverbindungsimplantat (1) nach einem der Ansprüche 1 bis 5, **dadurch gekennzeichnet, dass** das Knochenverbindungsimplantat (1) aus einem Metallwerkstoff hergestellt ist.

7. Knochenverbindungsimplantat (1) nach einem der Ansprüche 1 bis 6, **dadurch gekennzeichnet, dass** das Knochenverbindungsimplantat (1) zum Verbinden eines ersten Knochenbereiches (2) mit einem zweiten Knochenbereich (3) eines Oberkieferknochens (19) oder eines Unterkieferknochens vorbereitet ist.

8. Verfahren zum individualisierten Herstellen des Knochenverbindungsimplantats (1) nach einem der Ansprüche 1 bis 7, umfassend folgende Schritte:
a) Erfassen eines 3D-Ist-Modelles des Säugerknochens (4) in einem ersten Datensatz,
b) Erstellen eines 3D-Soll-Modelles in einem zweiten Datensatz unter Festlegen zwei Durchtrennungslinien (8, 17) an dem 3D-Ist-Modell sowie unter relativen Verschieben zweier imaginärer durch die zumindest eine Durchtrennungslinie (8, 17) getrennter Knochenbereiche (2, 3) relativ zueinander, und
c) Fertigen des Knochenverbindungsimplantats (1) anhand des 3D-Soll-Modelles, wobei der erste Befestigungsbereich (6) zur Befestigung an dem ersten Knochenbereich (2) des 3D-Soll-Modelles, der zweite Befestigungsbereich (7) zur Befestigung an dem zweiten Knochenbereich (3) des 3D-Soll-Modelles, der dritte Befestigungsbereich (16) zur Befestigung an dem erstem Knochenbereich (2) und die Trennwerkzeugführungskonturen (9, 18) unter einem zumindest teilweisen Nachbilden der zwei Durchtrennungslinien (8, 17) ausgeformt werden.

## Claims

1. A bone fusion implant (1) for fusing a first bone region (2) to a second bone region (3) severed from the first bone region (2) by osteotomy or a plurality of bone regions of a mammalian bone (4), comprising a first fixing region (6) which has multiple securing means receiving holes (5a) and is provided for attaching to the first bone region (2), and a second fixing region (7) which is connected to the first fixing region (6), wherein the second fixing region (7) in turn includes multiple securing means receiving holes (5b) and is provided for attaching to the second bone region (3), wherein between the first and second fixing regions (6, 7) a cutting tool guiding contour (9) specifying a severing line (8) is formed, wherein the cutting tool guiding contour (9) is formed by an inner edge (14a, 14b) of a frame structure (15a) configured between the first and second fixing regions (6, 7), **characterized in that** between the second fixing region (7) and a third fixing region (16) a second cutting tool guiding contour (18) specifying a severing line (17) is formed, wherein the third fixing region (16) in turn includes multiple securing means receiving holes (5c) and is provided for attaching to the first bone region (2).

2. Bone fusion implant (1) according to claim 1, **characterized in that** the cutting tool guiding contour (9) is formed on a connecting bar (10) associated with the first fixing region (6), wherein an underside of the connecting bar (10) is corrugated.

3. The bone fusion implant (1) according to claim 1 or 2, **characterized in that** the cutting tool guiding contour (9) is strip-shaped.

4. The bone fusion implant (1) according to one of the claims 1 to 3, **characterized in that** the cutting tool guiding contour (9) is formed directly by a connecting bar (10, 11) connected to the first fixing region (6) and/or the second fixing region (7).

5. The bone fusion implant (1) according to one of the claims 1 to 4, **characterized in that** the fixing regions (6, 7) and the cutting tool guiding contour (9) are formed of one piece of material.

6. The bone fusion implant (1) according to any one of the claims 1 to 5, **characterized in that** the bone fusion implant (1) is made from a metal material.

7. The bone fusion implant (1) according to any one of the claims 1 to 6, **characterized in that** the bone fusion implant (1) is provided for fusing a first bone region (2) to a second bone region (3) of a maxilla (19) or a mandible.

8. A method for the individualized production of the bone fusion implant (1) according to any one of the claims 1 to 7, comprising the following steps of:
a) recording an actual 3D model of the mammalian bone (4) in a first data set,
b) drafting a target 3D model in a second data set by determining two severing lines (8, 17) on the actual 3D model as well as by relatively moving two imaginary bone regions (2, 3) separated by the at least one severing line (8, 17) relative to each other, and
c) producing the bone fusion implant (1) by way of the target 3D model, wherein the first fixing region (6) is formed for fixing to the first bone region (2) of the target 3D model, the second fixing region (7) is formed for fixing to the second bone region (3) of the target 3D model, the third fixing region (16) is formed for fixing to the first bone region (2) and the cutting tool guiding contours (9) are formed by at least partially emulating the two severing lines (8, 17).

## Revendications

1. Implant de jonction osseuse (1) pour joindre une première zone osseuse (2) et une seconde zone osseuse (3) séparée par ostéotomie de la première zone osseuse (2) ou plusieurs zones osseuses d'un os de mammifère (4) avec une première zone de fixation (6) présentant plusieurs trous de logement de moyens de fixation (5a) préparée pour une application à la première zone osseuse (2), et une deuxième zone de fixation (7) reliée à la première zone de fixation (6), dans lequel la deuxième zone de fixation (7) présente à son tour plusieurs trous de logement de moyens de fixation (5b) et est préparée pour une application à la seconde zone osseuse (3), dans lequel un contour de guidage d'outil de coupe (9) définissant une ligne de sectionnement (8) est formé entre la première et la deuxième zone de fixation (6, 7), dans lequel le contour de guidage d'outil de coupe (9) est réalisé par un bord intérieur (14a, 14b) d'une structure de cadre (15a) conçue entre la première et la deuxième zone de fixation (6, 7), **caractérisé en ce qu'**un second contour de guidage d'outil de coupe (18) définissant une ligne de sectionnement (17) est formé entre la deuxième zone de fixation (7) et une troisième zone de fixation (16), dans lequel la troisième zone de fixation (16) présente à son tour plusieurs trous de logement de moyens de fixation (5c) et est préparée pour être appliquée à la première zone osseuse (2).

2. Implant de jonction osseuse (1) selon la revendication 1, **caractérisé en ce que** le contour de guidage d'outil de coupe (9) est formé sur une nervure de liaison (10) associée à la première zone de fixation (6), dans lequel une face inférieure de la nervure de liaison (10) est ondulée.

3. Implant de jonction osseuse (1) selon la revendication 1 ou 2, **caractérisé en ce que** le contour de guidage d'outil de coupe (9) est conçu en forme de barres.

4. Implant de jonction osseuse (1) selon l'une quelconque des revendications 1 à 3, **caractérisé en ce que** le contour de guidage d'outil de coupe (9) est formé directement par une nervure de liaison (10, 11) reliée à la première zone de fixation (6) et/ou la deuxième zone de fixation (7).

5. Implant de jonction osseuse (1) selon l'une quelconque des revendications 1 à 4, **caractérisé en ce que** les zones de fixation (6, 7) et le contour de guidage d'outil de coupe (9) sont réalisés en une seule pièce de matière.

6. Implant de jonction osseuse (1) selon l'une quelconque des revendications 1 à 5, **caractérisé en ce que** l'implant de jonction osseuse (1) est fabriqué à partir d'un matériau métallique.

7. Implant de jonction osseuse (1) selon l'une quelconque des revendications 1 à 6, **caractérisé en ce qu**e l'implant de jonction osseuse (1) est préparé pour joindre une première zone osseuse (2) et une seconde zone osseuse (3) d'un os maxillaire supérieur (19) ou d'un os maxillaire inférieur.

8. Procédé de fabrication individualisée de l'implant de jonction osseuse (1) selon l'une quelconque des revendications 1 à 7, comprenant les étapes suivantes consistant à :
a) détecter un modèle réel 3D de l'os de mammifère (4) dans un premier ensemble de données,
b) créer un modèle cible 3D dans un second ensemble de données en spécifiant deux lignes de sectionnement (8, 17) sur le modèle réel 3D et en déplaçant de manière relative deux zones osseuses imaginaires (2, 3) séparées l'une de l'autre par au moins une ligne de sectionnement (8, 17), et
c) fabriquer l'implant de jonction osseuse (1) à l'aide du modèle cible 3D, dans lequel la première zone de fixation (6) est formée pour une fixation à la première zone osseuse (2) du modèle cible 3D, la deuxième zone de fixation (7) est formée pour une fixation à la seconde zone osseuse (3) du modèle cible 3D, la troisième zone de fixation (16) est formée pour une fixation à la première zone osseuse (2) et les contours de guidage d'outil de coupe (9, 18) sont formés en reproduisant au moins partiellement les deux lignes de sectionnement (8, 17).
